# EUROPEAN PATENT APPLICATION

(11) **EP 3 821 800 A1**
(43) Date of publication of application: **19.05.2021**
(21) Application number: 19833043.3
(22) Date of filing: 12.07.2019
(51) Int. Cl.: A61B 5/0408, A61B 5/0478

(54) **GARMENT-TYPE ELECTRONIC APPARATUS AND METHOD OF MANUFACTURING SAME**

(30) Priority: 13.07.2018 JP 2018132974
(71) Applicant: TOYOBO CO., LTD., Osaka-shi Osaka 530-8230 (JP)
(72) Inventor: NARUSAWA Haruhiko, Otsu-shi, Shiga 520-0292 (JP); IRIE Michihiko, Otsu-shi, Shiga 520-0292 (JP); KONDO Takashi, Otsu-shi, Shiga 520-0292 (JP)
(74) Representative: Müller-Boré & Partner Patentanwälte PartG mbB
(86) International application number: PCT/JP2019/027745
(87) International publication number: WO 2020/013323

(57) **Abstract**

Provided is a garment for measuring biological information having favorable conductivity and stretchability that prevent a wiring and a bonding part from being disconnected by stress or deformation when the garment is put on, taken off, or washed, while detecting a stable electrical signal. In a wiring with a stretchable electrode according to an embodiment of the present invention, the electrode includes an electrically conductive sheet including electrically conductive fine particles and a binder resin, and the wiring incudes an electrically conductive fiber structure including an electrically conductive fiber. A part of a bond between the electrode and the wiring has a peeling strength of 0.5 to 20 N/cm.

## Description

### TECHNICAL FIELD

An embodiment of the present invention relates to a garment-type electronic device that measures a faint electrical signal in an electrical input or output that is transmitted between a living body and the electronic device, or gives an electrical stimulus to the living body as necessary. This measurement or stimulation is performed by allowing the electronic device to be in contact with a skin surface of the living body.

### BACKGROUND ART

For measurement of a faint bioelectrical signal inside a living body such as brainwaves, an electrocardiogram and an electromyogram, there have conventionally been an adhesive bioelectrode pad (Patent Document 1) or the like formed from a soft electrically conductive adhesive gel. Such an adhesive electrode pad has adhesiveness to skin and has been used for a subject at rest to establish electrical conductivity in such a manner as not to cause a noise or signal degradation in the bioelectrical signal.

For application to healthcare, disease prediction, or the like, a garment having a bioelectrode and a wiring has attracted attention. This bioelectrode contacts a body surface, and this wiring connects the electrode to a terminal to which a device for external transmission of biological information is connected. Such a garment enables continuous detection of a bioelectrical signal from a subject even in motion, while being worn for long hours in his/her daily life. Use of various materials or means for the electrode, the wiring, or the terminal has also been proposed.

An example of using a same material for the electrode and the wiring is a bioelectrical signal induction sensor (Patent Document 2) having a garment portion. In this sensor, an electrode uses an electrically conductive fiber-sewn section for detecting a bioelectrical signal, and a wiring uses an electrically conductive yarn including an electrically conductive fiber. Another example of using a same material for the electrode and the wiring is wear having a skin contact-type electrode with a wiring (Patent Document 3). These electrode and wiring use a fiber material containing an electrically conductive polymer or a sheet material formed from an electrically conductive composition containing an electrically conductive filler, such as metal powder, and a stretchable resin. This wear allows the electrode to be positioned on a certain place of a body surface, enabling measurement of biological information from a subject even during exercise.

Inversely, an example of using different materials for the electrode and the wiring is a garment having an electrode using an electrically conductive fiber structure and a wiring formed by printing an electrically conductive polymer or laminating an electrically conductive film (Patent Document 4). This electrically conductive fiber structure is a woven fabric, a knitted fabric, or a non-woven fabric including a fine metal wiring, a metal-plated fiber material, or a fiber material impregnated with an electrically conductive material. Another example of using different materials is a garment having a wiring and an electrode that uses an electrically conductive fiber, which is a fiber material coated with an electrically conductive polymer (Patent Document 5). In Example 5, the wiring is an electrically conductive ink, thus eliminating necessity of sewing an electrically conductive yarn into the garment. In Example 6, this garment has an electrically conductive hook-and-loop fastener, thus enabling a wearable device to be attached to any position. Yet another example of using different materials for the electrode and the wiring is an apparatus that is attached to a garment (Patent Document 6). Electrodes of this apparatus are metal terminals or use an electrically conductive fiber material sewn into the garment, and a wiring of this apparatus is an electrical wire or uses a spring-shaped electrically conductive fiber material. This apparatus senses with a posture detection sensor that either of the electrodes, which use the electrically conductive fiber material, comes into contact with skin and then compensates for a deficiency in adhesiveness of the electrodes to the skin, thus enabling detection of a biological signal.

### RELATED ART DOCUMENTS

### PATENT DOCUMENTS

Patent Document 1: JP-A-H09-215668
Patent Document 2: JP-A-2002-159458
Patent Document 3: JP-A-2017-029692
Patent Document 4: JP-A-2016-106877
Patent Document 5: JP-A-2016-136456
Patent Document 6: JP-A-2017-121442

### SUMMARY OF THE INVENTION

### TECHNICAL PROBLEM

When measurement of a bioelectrical signal is performed by wearing the garment having the wiring for connecting the electrode and the terminal, it is important for the electrode to have a larger area than the wiring, have a low surface resistance, and adhere to skin of a wearer of the garment even in motion without causing a shift in its position. On the other hand, it is necessary for the wiring to have stretchability close to a fabric of the garment to reduce a sense of discomfort felt by the wearer, and have a low specific resistance to prevent signal degradation. The wiring is also needed not to be disconnected by deformation stress, such as stretching, when the garment is put on, taken off, washed, dry-cleaned, or the like.

In Patent Document 2, the electrode and the wiring include the electrically conductive fiber. Such a fabric electrode using the electrically conductive fiber, however, is inferior in adhesiveness to skin owing to its interfiber void, and this inferior adhesiveness causes the electrode to shift when a subject moves. This shift might lead to failure in detecting a stable signal. Patent Document 3 exemplifies the electrode and the wiring that include the sheet material formed from the electrically conductive composition containing the electrically conductive filler, such as metal powder, and the stretchable resin. The sheet material has fewer voids than the fiber material and has superior adhesiveness to skin, but, for use as the wiring, an electrical bond of the electrically conductive filler is likely to be broken when the sheet material is stretched. This breakage might lead to disconnection of the wiring by deformation stress when the wear is put on, taken off, or washed.

In Patent Documents 4 and 5, in which different materials are used for the electrodes and the wirings, the electrodes include the electrically conductive fiber structure and thus are shifted when a subject moves. This shift might lead to failure in detecting a stable signal. Patent Document 6 exemplifies, for the electrodes, use of the metal terminal or the electrically conductive fiber material woven into the garment. This exemplification uses an additional sensor, such as the posture detection sensor, to compensate for a deficiency in adhesiveness of the electrodes to skin, posing a concern about an increase in cost. As described, properties required for the electrode and the wiring are different, and such a difference necessitates use of different materials that are suitable respectively for the electrode and the wiring. Using the different materials requires that a bond between the materials have a sufficient strength for rendering the electrode and the wiring appropriate for a garment for measuring biological information.

The present invention has been made with the above-mentioned technical problems as a backdrop. An object of the present invention is to provide a wiring with a stretchable electrode for a garment for measuring biological information, a method for producing the wiring with the stretchable electrode, and a garment for measuring biological information. The wiring with the electrode has a sufficient physical property and adhesiveness to a garment for preventing peeling of the electrode and disconnection of the wiring that are caused by stress or deformation when the garment is put on, taken off, or washed, while detecting a stable electrical signal. These sufficient physical property and adhesiveness are achieved by using the electrode having a favorable surface resistance and adhesiveness to skin and using the wiring having favorable electrical conductivity and stretchability even under elongational deformation.

### SOLUTIONS TO THE PROBLEMS

That is, an embodiment of the present invention has the following configuration.
[1] A garment-type electronic device comprising:
   an electrode configured to contact skin directly; and
   a stretchable electrical wiring,
   wherein the electrode comprises a stretchable electrically conductive sheet comprising electrically conductive fine particles and a binder resin,
   the stretchable wiring comprises an electrically conductive fiber structure.
[2] The garment-type electronic device according to above [1],
   wherein the electrically conductive fiber structure is one or more selected from the group consisting of a twisted yarn, a braid, a woven fabric, a knitted fabric, and a non-woven fabric that comprise an electrically conductive fiber.
[3] The garment-type electronic device according to above [1] or [2],
   wherein the electrode and the stretchable wiring are electrically bonded together, and
   the electrical bond between the electrode and the stretchable wiring is achieved by the electrically conductive fine particles coming into direct contact with the electrically conductive fiber.
[4] The garment-type electronic device according to any one of above [1] to [3],
   wherein a part of the electrical bond between the electrode and the stretchable wiring has a 90 degree peel strength of 0.5 to 20 N/cm.
[5] A method for producing the garment-type electronic device according to any one of above [1] to [4], comprising:
   (a) forming a stretchable wiring comprising an electrically conductive fiber structure on a garment substrate;
   (b) directly applying or printing an electrically conductive paste comprising electrically conductive fine particles and a binder resin in a pattern such that at least a part of the electrically conductive paste overlies the stretchable wiring; and
   (c) hardening the electrically conductive paste.
[6] A method for producing the garment-type electronic device according to any one of above [1] to [4], comprising:
   (d) obtaining an electrically conductive sheet by applying an electrically conductive paste comprising electrically conductive fine particles and a binder resin on a release substrate, and hardening the electrically conductive paste;
   (e) shaping the electrically conductive sheet into an electrode;
   (f) forming a stretchable wiring comprising an electrically conductive fiber structure on a garment substrate;
   (g) applying an electrically conductive paste comprising electrically conductive fine particles and a binder resin on a part of the stretchable wiring on which the electrode is positioned when the electrode is formed;
   (h) forming a thermoplastic resin layer in an electrode-forming position of the garment substrate excluding the part of the stretchable wiring on which the electrode is positioned when the electrode is formed; and
   (i) positioning the electrode-shaped electrically conductive sheet on the electrically conductive paste-applied part and in the electrode-forming position, and heating and pressing the electrode-shaped electrically conductive sheet.

### ADVANTAGEOUS EFFECTS OF THE INVENTION

The garment-type electronic device of the present invention has the electrode having stretchability that is configured to contact skin for measuring a biological signal and the stretchable wiring configured to connect the electrode and a terminal to which a device for external transmission of biological information is connected (Hereinafter, this terminal may be simply abbreviated to a terminal.). The stretchable electrode includes the electrically conductive sheet including the electrically conductive fine particles and the binder resin. This electrically conductive sheet is excellent in adhesiveness to skin and has a low surface resistance, thus enabling detection of a stable bioelectrical signal even when a subject wearing the garment-type electronic device is in motion. The stretchable wiring includes the electrically conductive fiber structure that has stretchability close to a fabric of the garment and a low specific resistance, and is not disconnected even by deformation stress, such as stretching. Such a stretchable wiring yields the garment-type electronic device for measuring biological information that significantly reduces a sense of discomfort felt by a wearer of the garment and can be handled as conveniently as an ordinary garment without being disconnected even when the garment is put on, taken off, dry-cleaned, or the like.

### BRIEF DESCRIPTION OF DRAWINGS

FIG. 1 is a schematic view (a cross-sectional view and a top view) showing a stretchable electrode and a stretchable wiring according to an embodiment of the present invention that are formed on a release PET film.
FIG. 2 is a schematic view (a cross-sectional view and a top view) showing a stretchable electrode, a stretchable wiring, and a connector according to an embodiment of the present invention that are formed on a clothing fabric.

The stretchable electrode and the stretchable wiring respectively include different materials. When an electrically conductive paste including a binder resin in which electrically conductive fine particles are dispersed is used for a bonding part between the stretchable electrode and the stretchable wiring, the electrically conductive paste permeates into an electrically conductive fiber structure in the stretchable wiring and exhibits a mechanical bonding strength with electrical conductivity retained. When an electrically conductive fiber is used for a bonding part between the stretchable electrode and the stretchable wiring, the electrically conductive fiber is sewn into the stretchable electrode and the stretchable wiring, thus exhibiting a mechanical bonding strength with electrical conductivity retained. The present invention becomes particularly effective when a distance between the stretchable electrode and a terminal to which a device for external transmission of biological information is connected is 15 cm or more and preferably 30 cm or more. Further preferably, the stretchable electrode and the terminal, to which a device for external transmission of biological information is connected, are provided across a seam of a fabric, for example provided on a front body section and a back body section of a garment, or provided on either of the body sections and a sleeve of a garment. A more specific example is to provide the electrode for electrocardiography on a chest portion and the device on a back neck portion; or to provide the electrode for electrocardiography on a chest portion and the terminal on a sleeve near a wrist.

### DESCRIPTION OF EMBODIMENTS

Hereinafter, a stretchable electrode and a stretchable wiring of the present invention are described. The stretchable electrode of the present invention that is configured to contact skin for measuring a biological signal includes an electrically conductive sheet including at least one kind of electrically conductive fine particles and at least one kind of a binder resin. The stretchable wiring of the present invention includes an electrically conductive fiber structure.

In an embodiment of the present invention, the electrically conductive fiber structure includes a fiber having electrical conductivity. In an embodiment of the present invention, the electrically conductive fiber structure may be a fiber structure including a fiber material at least including a yarn having electrical conductivity or the fiber having electrical conductivity. Also, in an embodiment of the present invention, the electrically conductive fiber structure may be a fiber structure having been subjected to a treatment in which electrical conductivity is imparted to the fiber structure having no electrical conductivity. Examples of the electrically conductive fiber structure according to an embodiment of the present invention include a woven fabric, a knitted fabric, and a non-woven fabric that include a fiber including an electrically conductive yarn (This yarn encompasses the yarn having electrical conductivity or the fiber having electrical conductivity, and the same will apply hereinafter.). The fiber structure according to an embodiment of the present invention preferably has a plane shape with a width and a length when the fiber structure is used as the stretchable wiring for a garment. The fiber structure is preferably a braid, a woven fabric, a knitted fabric, or a non-woven fabric. The fiber structure may also encompass a string or a yarn with a certain thickness or more because such a string or a yarn may be regarded as the stretchable wiring with a width and a length on a bonding surface with the electrode.

The electrically conductive yarn is a general term for an electrically conductive fiber, a fiber bundle of electrically conductive fibers, a twisted yarn, a plaited yarn, a spun yarn, and a blended yarn obtained from fibers including an electrically conductive fiber, a fine metal wiring obtained by finely stretching a metal wiring, and a fine film obtained by cutting a film into a fine fiber shape. In an embodiment of the present invention, examples of the electrically conductive fiber include a chemical fiber or a natural fiber coated with a metal, a chemical fiber or a natural fiber coated with an electrically conductive metal oxide, a chemical fiber or a natural fiber coated with a carbon-based electrically conductive material, and a chemical fiber or a natural fiber coated with an electrically conductive polymer. The chemical fiber is a regenerated fiber, such as rayon, cupro, and polynosic, a semisynthetic fiber, such as acetate, triacetate, and promix, or a synthetic fiber, such as nylon, aramid, vinylon, vinylidene, polyester, acrylic, polyethylene, polypropylene, polyurethane, and polylactic acid. One or more of these may be used as the chemical fiber. The natural fiber is a plant fiber, such as cotton and hemp, or an animal fiber, such as wool and silk. One or more of these may be used as the natural fiber. The electrically conductive fiber may be, for example, obtained by spinning a polymer material including at least one electrically conductive material selected from the group consisting of a metal, an electrically conductive metal oxide, a carbon-based electrically conductive material, and an electrically conductive polymer.

The metal is silver, gold, platinum, palladium, copper, nickel, aluminum, zinc, lead, tin, or the like, or an alloy, such as brass, bronze, cupronickel, and solder. The electrically conductive metal oxide is indium oxide, tin oxide, a zinc oxide metal, or the like. The carbon-based electrically conductive material is graphite, carbon, carbon nanotube, graphene, or the like. The electrically conductive polymer is polypyrrole, polyaniline, polythiophene, polyethylenedioxythiophene, poly (p-phenylene), poly (p-phenylene vinylene), polyfluorene, or polyacetylene. For the respective materials, one or more of these may be used.

The fiber bundle of electrically conductive fibers according to an embodiment of the present invention may be, for example, obtained by allowing a fiber bundle of micro fibers or nanofibers of the electrically conductive fiber to carry or be impregnated with an electrically conductive filler, an electrically conductive polymer, or the like.

The electrically conductive yarn according to an embodiment of the present invention may be the twisted yarn, the plaited yarn, the spun yarn, the blended yarn, or the like that is obtained from fibers including the electrically conductive fiber. The electrically conductive yarn also encompasses the fine metal wiring obtained by finely stretching a metal wiring. The electrically conductive fiber; the fiber bundle of the electrically conductive fibers; the twisted yarn, the plaited yarn, the spun yarn, and the blended yarn, which are obtained from the fibers including the electrically conductive fiber; and the fine metal wiring preferably have an average diameter of 250 µm or less. The average diameter is more preferably 120 µm or less, further preferably 80 µm or less, and particularly preferably 50 µm or less.

The electrically conductive yarn also encompasses the fine film obtained by cutting a film into a fine fiber shape. The fine film refers to a fibrous film obtained by cutting to a width of 800 µm or less a polymer film coated with at least one electrically conductive material selected from the group consisting of a metal, an electrically conductive metal oxide, a carbon-based electrically conductive material and an electrically conductive polymer. The fine film has the following elongation properties. A tensile elastic modulus is 500 MPa or less, and a load applied to the fine film when the wiring is stretched at a stretching rate of 10% is 100 N or less. When the fine film is used by being attached to the garment, these elongation properties allow the wiring to concomitantly stretch as a fabric of the garment is stretched. As a result, a fit of the garment is not spoilt.

The electrically conductive sheet in the present invention includes at least one kind of the electrically conductive fine particles and at least one kind of the binder resin. The electrically conductive fine particles are fine metal particles and/or carbon fine particles. The fine metal particles may be, for example, metal particles of silver, gold, platinum, palladium, copper, nickel, aluminum, zinc, lead, tin, or the like; alloy particles of brass, bronze, cupronickel, solder, or the like; hybrid particles like silver-coated copper; metal-plated polymer particles; metal-plated glass particles; metal-coated ceramic particles; or the like. The carbon fine particles may be graphite powder, activated carbon powder, scaly graphite powder, acetylene black, ketjen black, fullerene, a carbon nanotube, or the like. The electrically conductive fine particles of just one kind, or two or more kinds may be used.

The binder resin preferably has an elastic modulus of 1 GPa or less and an elongation at break of 200% or more. Examples of the binder resin include a thermoplastic resin, a thermosetting resin, a photocurable resin, and rubber or an elastomer. The thermoplastic resin may be low density polyethylene, an ethylene-vinyl acetate copolymer, polyvinylidene chloride, copolyester, or the like. The thermosetting resin or the photocurable resin may be an acrylic resin, a silicone resin, a polyurethane resin, or the like. Examples of the rubber or the elastomer include urethane rubber, acrylic rubber, silicone rubber, butadiene rubber, nitrile rubber, isoprene rubber, styrene butadiene rubber, butyl rubber, chlorosulfonated polyethylene rubber, ethylene propylene rubber, and a vinylidene fluoride copolymer. The binder resin of just one kind, or two or more kinds may be used. Amongst these, preference is given to the rubber or the elastomer for developing stretchability and adhesiveness to the electrically conductive fine particles.

The electrically conductive sheet may contain insulating fine particles to such an extent as not to impair necessary properties for exhibiting a mechanical property, heat resistance, durability and moisture permeability. The insulating fine particles are made of an organic or inorganic insulating material. The organic fine particles may be resin fine particles such as acrylic resin fine particles, styrene resin fine particles and melamine resin fine particles. The inorganic fine particles may be ceramic fine particles such as silica, alumina, zirconia, talc, silicon carbide, magnesia, boron nitride; or fine particles of a water-insoluble salt, such as calcium phosphate, magnesium phosphate, barium sulfate and calcium sulfate. The insulating fine particles of just one kind, or two or more kinds may be used.

A blending amount of the electrically conductive fine particles in the electrically conductive sheet of the present invention is determined, based upon surface resistance and stretchability. A high volume% of the electrically conductive fine particles relative to the binder resin decreases surface resistance, thus being able to prevent deterioration of an electrical signal. However, such a high volume% of the electrically conductive fine particles weakens an ability of the binder resin to retain the electrically conductive fine particles and deteriorates launderability and stretchability, thus worsening tightness and a fit. Inversely, a low volume% of the electrically conductive fine particles relative to the binder resin enhances stretchability and improves tightness and a fit. However, such a low volume% of the electrically conductive fine particles increases surface resistance, thus deteriorating an electrical signal. To keep a balance between both the properties, the blending amount of the electrically conductive fine particles relative to the binder resin is preferably 20 to 60 volume%.

The electrically conductive sheet has a tensile elastic modulus of 1 GPa or less, and a load applied to the electrically conductive sheet at a stretching rate of 10% is 200 N or less. When the tensile elastic modulus exceeds 1 GPa or the load applied to the electrically conductive sheet at the stretching rate of 10% exceeds 200 N, such an excessive tensile elastic modulus or an excessive load leads to a crack of the electrode that is caused by various postures or motions taken by a wearer of the garment, or on an occasion of his/her putting on, taking off, or washing the garment. This crack hinders the electrode from following skin upon measurement, resulting in noise contamination or deterioration of an electrical signal.

The electrically conductive sheet of the present invention is produced by a method of, for example, applying or printing an electrically conductive paste containing water or an organic solvent in addition to the electrically conductive fine particles and the binder resin. A content of the water or the organic solvent is determined, based, for example, upon a dispersion method of the electrically conductive fine particles, or upon viscosity and a drying method of the electrically conductive paste that are suitable for a production method of an electrically conductive film. The content affects a state of chain structure formation of the electrically conductive fine particles and moisture permeability of a film layer after the application. The content is preferably 20 to 60 mass% when total mass of the electrically conductive fine particles, the binder resin and the solvent is defined to be 100%, but is not particularly limited thereto. A content of less than 20 mass% leads to an increase in viscosity and thus is unsuitable for the application or the printing, causing uneven chain structure of the electrically conductive fine particles. Consequently, this uneven chain structure brings about deterioration of electrical conductivity and stretchability and a decrease in porosity in a film that results in low moisture permeability. Inversely, a content exceeding 60 mass% leads to an increase in a residual solvent amount in a dry-hardened coat and thus might cause deterioration of reliability of the coat.

The organic solvent preferably has a boiling point of from 100°C to less than 300°C. An organic solvent having an excessively low boiling point evaporates during a production step of the paste or upon use thereof and thus might be likely to cause variation in a component ratio of the paste. An organic solvent having an excessively high boiling point leads to an increase in a residual solvent amount in a dry-hardened coat and thus might cause reliability deterioration of the coat. Examples of the preferable organic solvent include cyclohexanone, toluene, isophorone, γ-butyrolactone, benzyl alcohol, ExxonMobil Chemical's solvesso 100, 150 and 200, propylene glycol monomethyl ether acetate, terpineol, butyl glycol acetate, diamylbenzene (boiling point: 260 to 280°C), triamylbenzene (boiling point: 300 to 320°C), n-dodecanol (boiling point: 255 to 29°C), diethylene glycol (boiling point: 245°C), ethylene glycol monoethyl ether acetate (boiling point: 145°C), diethylene glycol monoethyl ether acetate (boiling point 217°C), diethylene glycol monobutyl ether acetate (boiling point: 247°C), diethylene glycol dibutyl ether (boiling point: 255°C), diethylene glycol monoacetate (boiling point: 250°C), triethylene glycol diacetate (boiling point: 300°C), triethylene glycol (boiling point: 276°C), triethylene glycol monomethyl ether (boiling point: 249°C), triethylene glycol monoethyl ether (boiling point: 256°C), triethylene glycol monobutyl ether (boiling point: 271°C), tetraethylene glycol (boiling point: 327°C), tetraethylene glycol monobutyl ether (boiling point: 304°C), tripropylene glycol (boiling point: 267°C), tripropylene glycol monomethyl ether (boiling point: 243°C), 2,2,4-trimethyl-1,3-pentanediol monoisobutyrate (boiling point: 253°C), and two or more kinds of these organic solvents may be contained as necessary. Such an organic solvent is appropriately contained so that the electrically conductive paste has suitable viscosity for the application or the printing.

To impart application and printing properties, the electrically conductive paste for producing the electrically conductive sheet may be blended with a thixotropy imparting agent, a leveling agent, a plasticizer, an antifoaming agent, or the like to such an extent as not to impair necessary properties for a stretchable conductive film. The electrically conductive paste for forming the stretchable film layer having electrical conductivity according to an embodiment of the present invention can contain the electrically conductive fine particles dispersed evenly by a conventionally known method for dispersing fine particles in a solution. Such even dispersion can be achieved by, for example, an ultrasonic method, a mixer method, a three roll mill method, a ball mill method, or the like after mixture of a dispersion solution of the fine particles and a solution of the resin. These methods may also be adopted in combination of plural kinds.

In the wiring with the stretchable electrode according to an embodiment of the present invention, the stretchable electrode configured to contact skin for measuring a biological signal includes the electrically conductive sheet including the electrically conductive fine particles and the binder resin, and the stretchable wiring includes the electrically conductive fiber structure. Any one of the following methods may be adopted to electrically bond these materials of different kinds together and achieve a sufficient property for preventing the bonding part from being disconnected by stress or deformation when the garment is put on, taken off, or washed: (1) bonding the stretchable electrode and the stretchable wiring together with the electrically conductive paste including the binder resin in which the electrically conductive fine particles are dispersed; (2) forming the stretchable electrode and simultaneously bonding this stretchable electrode and the stretchable wiring by directly applying or printing the electrically conductive paste, which includes the binder resin in which the electrically conductive fine particles are dispersed, on a part of the stretchable wiring that is to contact the electrode; or (3) bonding the stretchable electrode and the stretchable wiring by sewing them together with the electrically conductive fiber. The method (1) may use the electrically conductive paste different from the electrically conductive paste used to form the stretchable electrode to such an extent as not to impair the necessary properties. The method (3) may also use the electrically conductive fiber different from the electrically conductive fiber included in the stretchable wiring to such an extent as not to impair the necessary properties.

In production of the electrically conductive sheet for the stretchable electrode of the present invention and production of the stretchable electrode and the wiring in the methods (1) and (2), the application of the electrically conductive paste may be performed by, for example, hands with a spatula, a coating method, a printing method, or the like, but is not particularly limited thereto. Examples of the printing method include a screen printing method, a planographic offset printing method, an inkjet method, a flexographic printing method, a gravure printing method, a gravure offset printing method, a stamping method, a dispensing method and a squeegee printing. A heating and drying step of the applied electrically conductive paste may be performed under an atmosphere, a vacuum atmosphere, an inert gas atmosphere, a reducing gas atmosphere, or the like. Such a heating and drying step evaporates the organic solvent and, in some cases, allows a hardening reaction to proceed during the heating, thus yielding favorable surface resistance and stretchability of a dried electrically conductive sheet. The heating and drying step under an atmosphere is performed at a heating temperature of 50 to 200°C and a heating time of 10 to 90 minutes. A condition for this heating and drying step is selected from either a low temperature-long time combination or a high temperature-short time combination, based upon surface resistance and stretchability of the electrically conductive sheet, heat resistance of the binder resin, a boiling point of the organic solvent, or the like. A heating temperature of less than 50°C or a heating time of less than 10 minutes causes the solvent to remain in the coat, thus failing to achieve desired surface resistance and stretchability. A heating temperature exceeding 200°C or a heating time exceeding 90 minutes causes deterioration and a cross-link in the binder resin, the flexible resin or a substrate, thus failing to achieve desired stretchability and leading to cost increase.

The wiring with the stretchable electrode according to an embodiment of the present invention, which includes the above materials and is formed by the above method, has an electrical bond between the stretchable electrode and the stretchable wiring, and a part of this electrical bond has a peeling strength of 0.5 to 20 N/cm. A peeling strength of less than 0.5 N/cm causes the part of the electrical bond to be disconnected by stress or deformation when the garment is put on, taken off, or washed. Apeeling strength of more than 20 N/cm exceeds a material strength of the stretchable electrode or the stretchable wiring, thus becoming meaningless in practical terms.

When the wiring with the stretchable electrode according to an embodiment of the present invention is applied to a fabric of a garment for measuring biological information, an insulating layer is needed to be formed in some cases. This insulating layer may be formed by coating a flexible resin or laminating a flexible resin sheet. The flexible resin for the insulating layer according to an embodiment of the present invention preferably has an elastic modulus of 1 GPa or less, and an elongation at break of 200% or more. Example of such a flexible resin include a thermoplastic resin, a thermosetting resin, a photocurable resin, and rubber or an elastomer. The thermoplastic resin may be low density polyethylene, an ethylene-vinyl acetate copolymer, polyvinylidene chloride, copolyester, or the like. The thermosetting resin or the photocurable resin may be an acrylic resin, a silicone resin, a polyurethane resin, or the like. Examples of the rubber or the elastomer include urethane rubber, acrylic rubber, silicone rubber, butadiene rubber, nitrile rubber, isoprene rubber, styrene butadiene rubber, butyl rubber, chlorosulfonated polyethylene rubber, ethylene propylene rubber, and a vinylidene fluoride copolymer. The binder resin of just one kind, or two or more kinds may be used.

The garment for measuring biological information according to an embodiment of the present invention includes the wiring with the stretchable electrode according to an embodiment of the present invention that is attached to a part inside the garment. A substrate of the garment for measuring biological information according to an embodiment of the present invention is any band-like object, such as a belt and a brassiere, and/or any garment made of a knitted fabric or a non-woven fabric without any particular limitation. The substrate is, however, preferably a stretchable object from a perspective of, for example, good attachability to a wearer for measurement of biological information, or of followability during exercise or in motion. Such a garment for measuring biological information becomes means to measure biological information of its wearer, is put on in a usual manner, and offers a usual fit. Hence, just wearing such a garment easily enables measurement of biological information of various kinds.

### EXAMPLES

Next, specific Examples of the present invention will be described. However, the present invention is not particularly limited to these Examples.

### [Production Example]

### <Production Example 1 of Electrically Conductive Yarn>

A nylon false twisted yarn (277 dtex) was silver-plated by an electroless plating method. A substrate treatment for the electroless silver plating was firstly performed as follows. The yarn was immersed in a scouring agent and washed with water. The water-washed yarn was then immersed in a solution containing 10 g/L of stannous chloride and 20 mL/L of 35% hydrochloric acid and washed with water to impart a catalytic property to the yarn. The resultant yarn, which had been subjected to this substrate treatment, was thereafter plated with 10 mass% of silver with a predetermined amount of an electroless silver plating solution having the following composition.

### [Electroless Silver Plating Bath Ratio] (5 g/L of Silver)

| | |
|---|---|
| Ethylenediaminetetraacetic acid tetrasodium | 100 g/L |
| Sodium hydroxide | 25 g/L |
| Formalin | 50 g/L |
| Silver nitrate (dissolved in 1 L of water and dropped) | 15.8 g |
| Ammonia water (dissolved in 1 L of water and dropped) | 50 mL |

The resultant silver-plated yarns were twisted together to produce an electrically conductive yarn (F1) having a resistance value of 120 mΩ per yarn length of 1 cm.

### < Production Example 2 of Electrically Conductive Yarn>

A filament of Belima X ultra-fine microfiber available from KB SEIREN, LTD. was immersed in an electrically conductive coating material Denatron including a thiophene-based electrically conductive polymer named PEDOT:PSS. The immersed filament was subjected to an ultrasonic treatment for 3 minutes and then pulled up while being squeezed. The resultant filament was dried to produce an electrically conductive yarn (F2) impregnated with the electrically conductive polymer. The produced electrically conductive yarn had a resistance value of 40 Ω per yarn length of 1 cm.

### <Production Example 1 of Electrically Conductive Paste>

Twenty parts by mass of Nitrile rubber Nipol DN003 available from Zeon Corporation was dissolved in 45 parts by mass of isophorone. The resultant resin solution was then blended with 60 parts by mass of agglomerated silver powder G-35 available from DOWA Electronics Materials Co., Ltd. and 20 parts by mass of flake-shaped silver powder FA-D-3 available from DOWA Electronics Materials Co., Ltd. The blended solution was mixed by a three roll mill to produce an electrically conductive paste (P1).

### < Production Example 2 of Electrically Conductive Paste>

Sixty five parts by mass of a polyester urethane resin solution Vylon UR6100 (solvent composition: cyclohexanone/solvesso 150/isophorone = 40/40/20 in a mass ratio) available from TOYOBO CO., LTD. was blended with 60 parts by mass of agglomerated silver powder G-35 available from DOWA Electronics Materials Co., Ltd. and 20 parts by mass of flake-shaped silver powder FA-D-3 available from DOWA Electronics Materials Co., Ltd. The blended solution was mixed by a three roll mill to produce an electrically conductive paste (P2).

### [Production of Electrically Conductive Fiber Structure]

Using the electrically conductive fiber produced in Production Example 1 and 330 dtex of a polyester false twisted yarn, a jersey fabric was knitted with a 22G single knitting machine. The electrically conductive fiber and the polyester false twisted yarn were interknitted together by being alternately fed (1×1) in such a manner as to have a knitted yarn length of 355 mm/100 W. The resultant grey fabric was scoured to finish the fabric with an appropriate width in order to produce an electrically conductive fiber structure (FF1). Properties of the produced electrically conductive fiber structure are shown in Table 1.

Using the electrically conductive fiber produced in Production Example 2 and 110 dtex of a polyester false twisted yarn, a circular rib fabric was knitted with an 18 G interlock knitting machine. The electrically conductive fiber and the polyester false twisted yarn were interknitted together by being alternately fed (1×1) in such a manner as to have a knitted yarn length of 450 mm/100 W. The resultant grey fabric was scoured to finish the fabric with an appropriate width in order to produce an electrically conductive fiber structure (FF2). Properties of the produced electrically conductive fiber structure are shown in Table 1.

### [Production of Electrically Conductive Sheet]

The electrically conductive pastes produced in Production Examples 1 and 2 were respectively applied on release PET films with an applicator such that dried film thicknesses of the electrically conductive pastes were to become approximately 120 µm. The resultant films were then dried in a hot air drying oven at 120°C for 30 minutes. Thereafter, the release PET films were peeled to produce electrically conductive sheets (PS1) and (PS2). Properties of the produced electrically conductive sheets are shown in Table 1.

**[Table 1]**

| Electrically Conductive Fabric/Electrically Conductive Sheet | FF1 | FF2 | PS1 | PS2 |
|---|---|---|---|---|
| Electrically Conductive Yarn/Electrically Conductive Paste | F1 | F2 | P1 | P2 |
| Thickness (µm) | 700 | 720 | 126 | 72 |
| Mass Per Unit Area (g/m²) | 179 | 140 | 450 | 250 |
| Tensile Elastic Modulus (MPa) | 280 | 690 | 141 | 155 |
| Applied Load Upon 10% Stretching (N/cm) | 37 | 30 | 82 | 85 |

### [Production of Wiring with Stretchable Electrode]

The electrically conductive fiber structure and the electrically conductive sheet, which were produced above, were cut into a circular shape with a diameter of 5.0 cm to obtain a stretchable electrode and into a rectangular shape with a width of 1.0 cm and a length of 16.0 cm to obtain a stretchable wiring. Then, approximately 0.05 g of the electrically conductive paste was applied to a part of approximately 1.0 square cm of an edge part of the circular electrode. Onto this paste-applied part, an edge part of 1.0 cm of the rectangular wiring was superposed and then held together with 2 clips. This clip-held part was dried in a hot air drying oven at 120°C for 30 minutes to produce a wiring with a stretchable electrode that were bonded with the electrically conductive paste.

### [Production Example 1 of Garment-type Electronic Device (Example 1)]

As a garment substrate, a stretchable double raschel knitted fabric was used. To a predetermined position of the substrate, a stretchable wiring including the electrically conductive fiber structure (FF1) was firstly stuck with a hot melt sheet (a polyurethane hot melt adhesive, ELPHAN UH available from Nihon Matai Co., Ltd.) with a hot press. Subsequently, the electrically conductive paste (P1) was screen-printed such that the electrically conductive paste (P1) was to overlie a part of the stretchable wiring that was 1 cm from an edge thereof and to have a flat-part thickness of 35 µm. The printed paste was subjected to a predetermined drying process to obtain a substrate having an electrically conductive trace in which an electrode and the wiring were electrically bonded together.

To observe a bonding state between the stretchable wiring and the electrode, a cross-section of a bonding part therebetween was observed with a microscope. The observation showed that electrically conductive fine particles in the electrically conductive paste was in direct contact with the electrically conductive fiber.

The obtained substrate having the electrically conductive trace was cut according to a paper pattern to sew a men's compression shirt having symmetrically on its whole chest portion the electrodes for electrocardiography. In addition, to a predetermined position of the stretchable wiring, a stainless-steel snap hook was attached such that the stretchable wiring and a base of the snap hook were in direct contact with one another. With this snap hook being as a connector, a heart rate sensor WHS-2 available from UNION TOOL Co. was connected to the snap hook to produce 10 shirts into which heart rate measuring functions were respectively integrated (TS01).

From the produced shirts (TC01), 6 shirts were arbitrarily selected, and a CN ratio of electrocardiography was evaluated according to the following evaluation methods. The result showed that any of these 6 shirts were capable of electrocardiography with a favorable SN ratio. From these 6 shirts, 3 shirts were arbitrarily selected, and from these selected shirts, the electrodes and their adjacent 20 cm square portions were cut out. The shirt-substrate side of the cut portion with the electrode was stuck to a horizontal part of a 90 degree peeling jig, using a double sided carpet tape. Then, a stretchable electrode layer of a part at which the stretchable electrode overlay the stretchable wiring was notched with a razor. A gummed tape, which had been cut to overlie the stretchable electrode, was stuck to the stretchable electrode. The stretchable electrode together with the gummed tape were held with an upper clip of a tensile tester and peeled in a vertical direction to measure a 90 degree peel strength between the stretchable wiring and the stretchable electrode. An N3 average of the measured peeling strengths was 6.3 N/cm.

The remaining 3 shirts were subjected to a washing test. Even after the washing test, the 3 shirts were capable of electrocardiography in a same manner as before the washing test, and no deterioration of their performance was observed. For each of these 3 shirts, which had been subjected to this washing test, a 90 degree peel strength of the stretchable wiring and the stretchable electrode was measured in the same manner as above. An N3 average of the measured peeling strengths was 5.7 N/cm, and no significant deterioration was observed.

### [SN Ratio of Electrocardiography]

As subjects, 3 male adults wore the shirts with the heart rate measuring functions, which were produced in the above Example, and performed radio calisthenics Nos. 1 and 2 consecutively, while electrocardiography was being performed at 15°C and a RH of 40%. From a voltage waveform of the electrocardiogram excluding the first and last 1 minute of calisthenics time, a variance of amplitudes of R waves was defined as a signal (S), and a variance of amplitudes between the R waves was defined as a noise (N). From the expression of S/N, an SN ratio was calculated.

### [Washing Test]

The above-produced shirt with the electrode was washed by a method in accordance with JIS L 0844. The shirt in a laundry net was consecutively washed 5 times with a detergent (Attack available from Kao Corporation) in a mechanical washing machine, and then was once dried in shade. This process was defined as 1 cycle, and this cycle was repeated 10 times. After the washing, an SN ratio of electrocardiography was calculated in the same manner as above, using the washed garment for measuring biological information having the wiring with the stretchable electrode.

### [Production Example 2 of Garment-type Electronic Device (Example 2)]

Ten shirts into which heart rate measuring functions were respectively integrated (TS02) were produced in the same manner as in Production Example 1 of Garment-type Electronic Device except that the stretchable wiring was changed to a wiring including (FF2), and the same evaluations were performed. The evaluations showed favorable results like Production Example 1 of Garment-type Electronic Device. An initial N3 average of 90 degree peel strengths between the electrodes and the stretchable wirings was 4.3 N/cm, and an N3 average after the washing test was 4.2 N/cm.

### [Production Examples 3 and 4 of Garment-type Electronic Device (Examples 3 and 4)]

Shirts into which heart rate measuring functions were respectively integrated (TS03) were produced in the same manner as in Production Example 1 of Garment-type Electronic Device except that the electrically conductive paste was changed to PS2. Likewise, shirts (TC04) were produced with the stretchable wiring being changed to (FF2). The produced shirts were evaluated in the same manner, and the results of the evaluations for any of these shirts were favorable like Production Example 1 of Garment-type Electronic Device. In Production Example 3, an N3 average of 90 degree peel strengths between the electrodes and the stretchable wirings was initially 5.2 N/cm, and 4.8 N/cm after the washing test. In Production Example 4, an N3 average was initially 5.7 N/cm, and 5.5 N/cm after the washing test.

### [Production Example 5 of Garment-type Electronic Device (Example 5)]

As a substrate, a stretchable double raschel knitted fabric was used. On a predetermined position of the substrate, a stretchable wiring was formed by embroidering zigzag stitches on the substrate with the electrically conductive yarn F1. Subsequently, the electrically conductive paste (P1) was screen-printed such that the electrically conductive paste (P1) was to overlie a part of the stretchable wiring that was 1 cm from an edge thereof and to have a flat-part thickness of 20 µm. The printed paste was subjected to a predetermined drying process to obtain a substrate having an electrically conductive trace in which an electrode and the wiring were electrically bonded together.

To observe a bonding state between the stretchable wiring and the electrode, a cross-section of a bonding part therebetween was observed with a microscope. The observation showed that electrically conductive fine particles in the electrically conductive paste was in direct contact with the electrically conductive fiber.

The obtained substrate having the electrically conductive trace was cut according to a paper pattern to sew a men's compression shirt having symmetrically on its whole chest portion the electrodes for electrocardiography. In addition, to a predetermined position of the stretchable wiring, a stainless-steel snap hook was attached such that the stretchable wiring and a base of the snap hook were in direct contact with one another. With this snap hook being as a connector, a heart rate sensor WHS-2 available from UNION TOOL Co. was connected to the snap hook to produce 10 shirts into which heart rate measuring functions were respectively integrated (TS05). The same evaluations were performed on the produced shirts, and the results of the evaluations were favorable like Production Example 1 of Garment-type Electronic Device. An N3 average of 90 degree peel strengths between the electrodes and the stretchable wirings was initially 6.4 N/cm, and 6.1 N/cm after the washing test.

### [Production Example 6 of Garment-type Electronic Device (Example 6)]

Shirts into which heart rate measuring functions were respectively integrated (TS06) were produced in the same manner as in Production Example 5 of Garment-type Electronic Device except that the electrically conductive yarn was changed to F2 and the electrically conductive paste was changed to P2. The same evaluations were performed on the produced shirts, and the results of the evaluations were favorable like Production Example 1 of Garment-type Electronic Device. An N3 average of 90 degree peel strengths between the electrodes and the stretchable wirings was initially 6.2 N/cm, and 5.9 N/cm after the washing test.

### [Production of Electrode Component and Stretchable Wiring Component]

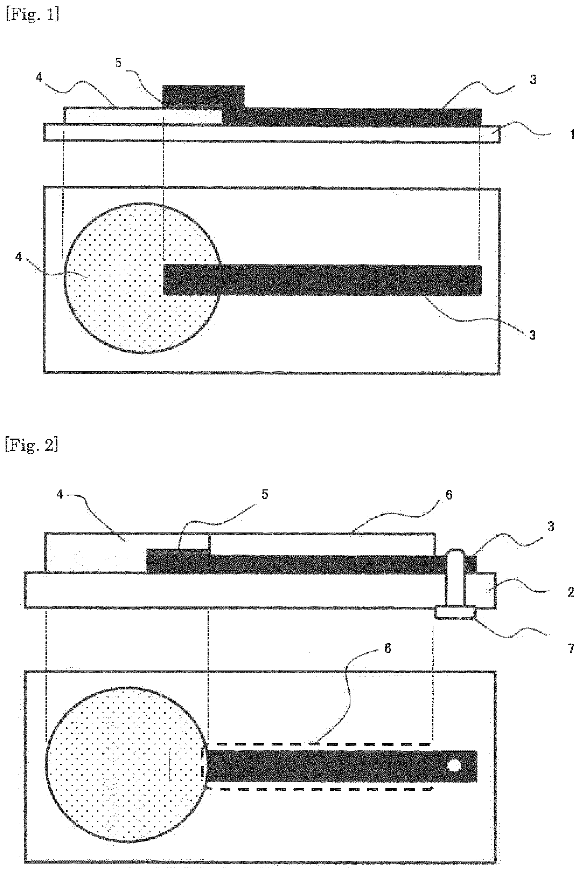

The electrically conductive fiber structure and the electrically conductive sheet were cut into a circular shape with a diameter of 5.0 cm to produce a stretchable electrode component and into a rectangular shape with a width of 1.0 cm and a length of 16.0 cm to produce a stretchable wiring component. Then, approximately 0.05 g of the electrically conductive paste (P1) was applied to a part of approximately 1.0 square cm of an edge part of the electrode component that corresponded to a bonding part with a wiring. Onto this paste-applied part, an edge part of 1.0 cm of the stretchable wiring component was superposed and then held together with 2 clips. This clip-held part was dried in a hot air drying oven at 120°C for 30 minutes to obtain an electrically conductive trace in which a stretchable electrode and a stretchable wiring were bonded together with the electrically conductive paste, as shown in FIG. 1. Properties of the obtained electrically conductive trace are shown in Table 2.

### [Production Example 7 of Garment-type Electronic Device (Example 7)]

A hot melt sheet (a polyurethane hot melt adhesive, ELPHAN UH available from Nihon Matai Co., Ltd.) was cut into a rectangular shape with a width of 1.0 cm and a length of 15.0 cm and into a circular shape with a diameter of 5.0 cm that was contiguous with the rectangular-shaped sheet. The resultant sheet was placed on a release PET film, and onto this sheet, the above-produced electrically conductive trace was superposed. Onto a part of the stretchable wiring corresponding to an insulating cover, a hot melt sheet, which had been cut into a rectangular shape with a width of 1.2 cm and a length of 15 cm, was superposed. On this rectangular-shaped sheet, a release PET film was placed, and the resultant laminate was thermocompression bonded with a roll laminating machine whose rubber roll temperature was adjusted to be 120°C in order to obtain an electrically conductive trace component having the release PET films and the hot melt sheet layers. The release PET film on one side of the electrically conductive trace component was peeled off, and the electrically conductive trace component was disposed in a predetermined position inside a shirt (a 100% polyester knit fabric) and thermocompression bonded with an iron. The release PET film on the other side was peeled off to produce a shirt with the electrode inside.

To a predetermined position of the produced shirt, a stainless-steel hook was attached to obtain a shirt having the stretchable electrode and the stretchable wiring, as shown in FIG. 2. To this hook, a heart rate sensor WHS-2 available from UNION TOOL Co. was connected to obtain a shirt TS07 into which a heart rate measuring function was integrated.

Based upon the combinations in Table 2, electrically conductive traces were produced likewise and then evaluated according to predetermined methods. The evaluation results are shown in Table 2.

The properties shown in Tables 1 and 2 were measured in the following methods.

### [Measurement of Thickness, Mass Per Unit Area, Tensile Elastic Modulus, and Load Applied upon 10% Stretching]

A thickness of the electrically conductive sheet was measured in accordance with JIS K7130 (1999), Plastics-Film and sheeting-Determination of thickness, Method A. Mass per unit area of the electrically conductive fiber structure was measured in accordance with JIS L1096 (2010), Testing methods for woven and knitted fabrics, 8.3 Mass per unit area. A tensile elastic modulus of the sheet and a load applied to the sheet upon stretching were measured in accordance with JIS K7161-1 (2012), Plastics-Determination of tensile properties-Part 1. Measurement conditions were as follows: a specimen width of 1.5 cm, a specimen length of 15 cm, a distance between chucks of 10 cm, and a test speed of 5.0 cm/min.

### [Measurement of Peeling Strength in Table 2]

Apeeling strength was measured in accordance with JIS L1086 (2013), Testing methods for fusible interlining fabrics and laminated fabrics, 7.10 Peeling strength.

The electrically conductive fiber structure and the electrically conductive sheet that had been produced were cut into tapes with a width of 2.5 cm and a length of 15 cm to obtain specimens. To a part of one specimen that was within 5 cm from an edge thereof, approximately 0.25 g of the above electrically conductive paste was applied, and the other specimen was superposed onto this paste-applied part. This bonded part of the specimens was held with 2 clips and dried in a hot air drying oven at 120°C for 30 minutes to produce specimens for measurement that were bonded together with the electrically conductive paste. Upper edges of these specimens were respectively held with an upper chuck and a lower chuck of a tensile tester (available from ORIENTEC CORPORATION) such that a distance between the chucks became 5.0 cm. The specimens were peeled by 5 cm at a stretching speed of 10.0 cm/min, and strengths that were required during this 5 cm peeling were measured. An average value of the measured strengths was defined as the peeling strength.

[SN Ratio of Electrocardiography] and [SN Ratio After Washing] were evaluated in the same manner as in [Production Example 1 of Garment-type Electronic Device (Example 1)]. In Comparative Examples 1 and 3, the SN ratios were deteriorated after the washing, and a crack in a wiring part of the electrically conductive sheet was seen through a visual observation. This crack was supposedly caused during the washing and brought about deterioration of a function as a wiring. In Comparative Example 2, the bonding part was disconnected when the shirt was put on or taken off, and owing to this disconnection, electrocardiography could not be performed.

**[Table 2]**

| Production Example | | Production Example 7 | Production Example 8 | Production Example 9 | Production Example 10 | Production Example 11 | Production Example 12 | Production Example 13 |
|---|---|---|---|---|---|---|---|---|
| | | Example | | | | Comparative Example | | |
| Composition of Electrically Conductive Trace | Electrode | PS1 | PS1 | PS2 | PS2 | PS1 | FF1 | FF2 |
| | Wiring | FF1 | FF2 | FF1 | FF2 | PS2 | FF2 | PS1 |
| | Bonding Paste | P1 | P2 | P1 | P2 | P1 | P2 | P2 |
| Electrode/Wiring Peeling Strength | (N/cm) | 2.24 | 1.21 | 1.35 | 1.73 | 1.18 | 0.34 | 1.05 |
| Shirt with Electrode | Possibility of Electrocardiography | Possible | Possible | Possible | Possible | Possible | Impossible | Possible |
| | SN Ratio | 32,1 | 28.0 | 26.4 | 22.6 | 5.8 | - | 3.7 |
| | SN Ratio After Washing | 81.7 | 27.7 | 25.8 | 21.4 | 4.2 | - | 2.4 |

### INDUSTRIAL APPLICABILITY

As mentioned above, an embodiment of the present invention provides a wiring with a stretchable electrode for measuring biological information and a garment for measuring biological information. The stretchable electrode and the stretchable wiring include materials that are respectively suitable therefor, and are bonded together with a sufficient strength for preventing breakage of a bonding part between the stretchable wiring and the stretchable electrode. This prevention enables the garment to withstand stress that is caused when the garment is put on, taken off, or washed, and consequently enables measurement of a favorable electrical signal. The wiring with the stretchable electrode and the garment are applicable to healthcare in a daily life, to a check on biological information during outdoor sports, such as jogging and a marathon, and to labor management of outdoor work on a construction site or the like. Also, an embodiment of the present invention can be used not only for measurement of a biological signal but also for a device that gives an electrical stimulus to a muscle to make the muscle move.

### REFERENCE SIGNS LIST

- 1.: release PET film
- 2.: garment substrate
- 3.: stretchable wiring
- 4.: stretchable electrode
- 5.: electrically conductive adhesive
- 6.: insulating cover
- 7.: connector

## Claims

1. A garment-type electronic device comprising:
an electrode configured to contact skin directly; and
a stretchable electrical wiring,
wherein the electrode comprises a stretchable electrically conductive sheet comprising electrically conductive fine particles and a binder resin,
the stretchable wiring comprises an electrically conductive fiber structure.

2. The garment-type electronic device according to claim 1,
wherein the electrically conductive fiber structure is one or more selected from the group consisting of a twisted yarn, a braid, a woven fabric, a knitted fabric, and a non-woven fabric that comprise an electrically conductive fiber.

3. The garment-type electronic device according to claim 1 or 2,
wherein the electrode and the stretchable wiring are electrically bonded together, and
the electrical bond between the electrode and the stretchable wiring is achieved by the electrically conductive fine particles coming into direct contact with the electrically conductive fiber.

4. The garment-type electronic device according to any one of claims 1 to 3,
wherein a part of the electrical bond between the electrode and the stretchable wiring has a 90 degree peel strength of 0.5 to 20 N/cm.

5. A method for producing the garment-type electronic device according to any one of claims 1 to 4, comprising:
(a) forming a stretchable wiring comprising an electrically conductive fiber structure on a substrate;
(b) directly applying or printing an electrically conductive paste comprising electrically conductive fine particles and a binder resin in a pattern such that at least a part of the electrically conductive paste overlies the stretchable wiring; and
(c) hardening the electrically conductive paste.

6. A method for producing the garment-type electronic device according to any one of claims 1 to 4, comprising:
(d) obtaining an electrically conductive sheet by applying an electrically conductive paste comprising electrically conductive fine particles and a binder resin on a release substrate, and hardening the electrically conductive paste;
(e) shaping the electrically conductive sheet into an electrode;
(f) forming a stretchable wiring comprising an electrically conductive fiber structure on a garment substrate;
(g) applying an electrically conductive paste comprising electrically conductive fine particles and a binder resin on a part of the stretchable wiring on which the electrode is positioned when the electrode is formed;
(h) forming a thermoplastic resin layer in an electrode-forming position of the garment substrate excluding the part of the stretchable wiring on which the electrode is positioned when the electrode is formed; and
(i) positioning the electrode-shaped electrically conductive sheet on the electrically conductive paste-applied part and in the electrode-forming position, and heating and pressing the electrode-shaped electrically conductive sheet.
